# EUROPEAN PATENT SPECIFICATION

(11) **EP 3 349 643 B1**
(45) Date of publication and mention of the grant of the patent: **17.02.2021**
(21) Application number: 16849467.2
(22) Date of filing: 21.09.2016
(51) Int. Cl.: A61B 3/08

(54) **METHODS, SYSTEMS AND COMPUTER PROGRAM PRODUCTS FOR DETERMINING OBJECT DISTANCES AND TARGET DIMENSIONS USING LIGHT EMITTERS**
VERFAHREN, SYSTEME UND COMPUTERPROGRAMMPRODUKTE ZUR BESTIMMUNG VON OBJEKTABSTÄNDEN UND ZIELABMESSUNGEN ANHAND VON LICHTEMITTERN
PROCÉDÉS, SYSTÈMES ET PRODUITS DE PROGRAMME INFORMATIQUE POUR DÉTERMINER DES DISTANCES D'OBJET ET DES DIMENSIONS DE CIBLE AU MOYEN D'ÉMETTEURS DE LUMIÈRE

(30) Priority: 23.09.2015 US 201562222273 P
(43) Date of publication of application: 25.07.2018
(73) Proprietor: East Carolina University, Greenville, North Carolina 27858 (US)
(72) Inventor: CHEN, Cheng, Greenville, North Carolina 27858 (US); PENG, Zhiyong, Greenville, North Carolina 27858 (US); JACOBS, Kenneth Michael, Greenville, North Carolina 27834 (US); FERGUSON, Bruce T. Jr., Raleigh, NC 27615 (US)
(74) Representative: Yeadon IP Limited
(86) International application number: PCT/US2016/052788
(87) International publication number: WO 2017/053368

(56) References cited:
- EP-A2- 2 524 650
- US-A1- 2008 049 268
- US-A1- 2009 041 201
- US-A1- 2010 209 002
- US-A1- 2014 003 740
- US-A1- 2014 276 097

## Description

### FIELD

The present inventive concept relates generally to tissue and organ blood flow and perfusion imaging and, more particularly, to determining target distances during imaging with large field of view and illumination.

### BACKGROUND

During the imaging process, the distance from the target (sample) to the camera lens needs to be within a certain range to ensure quality of the image, sufficient illumination and size of the field of view (FOV). This is distance is referred to herein as the "object distance." In some imaging applications, an approximate dimension of the target needs to be estimated without contacting the sample. Imaging applications include both surgical imaging and clinical imaging for in-patient as well as out-patient procedures.

Some systems use a near infra-red distance sensor to obtain the proper object distance. However, in these systems, the distance information needs to feedback to a computer continuously in real time, which increases the complexity of the software algorithm. Furthermore, the cost of near infra-red distance sensors is relatively high. Although, ultrasonic distance sensors tend to be cheaper than near infra-red sensors, they are also less accurate.

Furthermore, during an imaging procedure knowing the dimension of the target tissue/organ (target region) is an advantage. Some systems provide a surgical/clinical ruler that can placed beside the target. The target is imaged with the rule beside it, thus, revealing the approximate dimension of the target region. This solution typically requires contact with the tissue/organ and may increase the complexity and duration of the procedure. Furthermore, when imaged, ticks of the ruler placed beside the target might not be visible or clear in near infra-red image. Accordingly, improved systems of determining object distance and/or target dimensions may be desired.

US 2014/0276097 A1 describes a system for performing optical measurements for in-situ surgical applications. EP 2 524 650 A2 describes a method utilizing triangulation for in-situ surgical applications.

### SUMMARY

There is provided a method according to claim 1 and a system according to claim 11. Some embodiments of the present inventive concept provide methods for determining parameters during a clinical procedure, the methods including projecting a first pattern from a light emitter onto an object plane associated with a target to be imaged; projecting a second pattern from the light emitter onto the object plane associated with the target to be imaged; and manipulating the first and second patterns such that the first and second patterns overlap at one of a common point, line or other geometry indicating a proper object distance from the target to be imaged.

In further embodiments, projecting the first and second patterns may include projecting the first and second patterns having marks indicating a unit of measure indicating dimensions of the target to be imaged. The units of measure may function in at least two dimensions.

In still further embodiments, projecting the first and second patterns may include projecting first and second crosshair patterns onto the object plane, each of the crosshair patterns having tick marks on the axes indicating a unit of measure.

In some embodiments, manipulating may further include manipulating the first and second crosshair patterns such that center points of each directly overlap indicating the proper object distance from the target.

In further embodiments, the method maybe performed during one of a clinical and/or surgical imaging procedure in real time.

In still further embodiments, the light emitter may be a first light emitter that projects the first pattern onto the object plane and a second light emitter that projects the second pattern onto the object plane.

In some embodiments, projecting may further include projecting the first and second patterns onto the object plane using first and second laser emitters, respectively, each having wavelengths of from about 350nm to about 1000nm.

In further embodiments, the method may be non-invasive and performed in real time.

In some embodiments, the object distance may be a distance from the target to a camera lens.

In further embodiments, a wavelength of the emitters and corresponding patterns may be selected such that functionality is not affected ambient light.

Still further embodiments provide related systems and computer program products.

### BRIEF DESCRIPTION OF THE DRAWINGS

Figure 1 is a diagram of an imaging system using techniques in accordance with some embodiments of the present inventive concept.
Figures 2A and 2B are cross hair patterns that are projected on a surface plane with the target region where the tick marks/numbers indicate dimensions in accordance with some embodiments of the present inventive concept.
Figure 3 is a diagram illustrating overlapping cross hair patterns projected on a target region that illustrate an incorrect object distance in accordance with some embodiments of the present inventive concept.
Figure 4 is a diagram illustrating overlapping cross hair patterns projected on a target region at a proper object distance in accordance with some embodiments of the present inventive concept.
Figure 5 is a flowchart illustrating operations of a system in accordance with some embodiments of the present inventive concept.
Figures 6A and 6B are horizontal (A) and vertical (B) patterns generated by light emitters in accordance with some embodiments of the present inventive concept.
Figure 7 is an image illustrating a correct target distance in accordance with some embodiments of the present inventive concept
Figure 8 is an image illustrating a target distance that is not right in accordance with some embodiments of the present inventive concept.
Figure 9 is an image illustrating embodiments with a room light on and a light emitting diode (LED) light off in accordance with some embodiments of the present inventive concept.
Figure 10 is an image illustrating embodiments with both a room light on and an LED light on in accordance with some embodiments of the present inventive concept.

### DETAILED DESCRIPTION OF EMBODIMENTS

Embodiments of the present inventive concept will now be described more fully hereinafter with reference to the accompanying figures, in which some embodiments of the inventive concept are shown. This inventive concept may, however, be embodied in many different forms and should not be construed as limited to the embodiments set forth herein. Like numbers refer to like elements throughout. In the figures, layers, regions, elements or components may be exaggerated for clarity. Broken lines illustrate optional features or operations unless specified otherwise.

The terminology used herein is for the purpose of describing particular embodiments only and is not intended to be limiting of the inventive concept. As used herein, the singular forms "a", "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof. As used herein, the term "and/or" includes any and all combinations of one or more of the associated listed items. As used herein, phrases such as "between X and Y" and "between about X and Y" should be interpreted to include X and Y. As used herein, phrases such as "between about X and Y" mean "between about X and about Y." As used herein, phrases such as "from about X to Y" mean "from about X to about Y."

Unless otherwise defined, all terms (including technical and scientific terms) used herein have the same meaning as commonly understood by one of ordinary skill in the art to which this inventive concept belongs. It will be further understood that terms, such as those defined in commonly used dictionaries, should be interpreted as having a meaning that is consistent with their meaning in the context of the specification and relevant art and should not be interpreted in an idealized or overly formal sense unless expressly so defined herein. Well-known functions or constructions may not be described in detail for brevity and/or clarity.

It will be understood that when an element is referred to as being "on", "attached" to, "connected" to, "coupled" with, "contacting", etc., another element, it can be directly on, attached to, connected to, coupled with or contacting the other element or intervening elements may also be present. In contrast, when an element is referred to as being, for example, "directly on", "directly attached" to, "directly connected" to, "directly coupled" with or "directly contacting" another element, there are no intervening elements present. It will also be appreciated by those of skill in the art that references to a structure or feature that is disposed "adjacent" another feature may have portions that overlap or underlie the adjacent feature.

It will be understood that, although the terms first, second, etc. may be used herein to describe various elements, components, regions, layers and/or sections, these elements, components, regions, layers and/or sections should not be limited by these terms. These terms are only used to distinguish one element, component, region, layer or section from another element, component, region, layer or section. Thus, a first element, component, region, layer or section discussed below could be termed a second element, component, region, layer or section without departing from the teachings of the inventive concept. The sequence of operations (or steps) is not limited to the order presented in the claims or figures unless specifically indicated otherwise.

Spatially relative terms, such as "under", "below", "lower", "over", "upper" and the like, may be used herein for ease of description to describe one element or feature's relationship to another element(s) or feature(s) as illustrated in the figures. It will be understood that the spatially relative terms are intended to encompass different orientations of the device in use or operation in addition to the orientation depicted in the figures. For example, if a device in the figures is inverted, elements described as "under" or "beneath" other elements or features would then be oriented "over" the other elements or features. Thus, the exemplary term "under" can encompass both an orientation of over and under. The device may be otherwise oriented (rotated 90 degrees or at other orientations) and the spatially relative descriptors used herein interpreted accordingly. Similarly, the terms "upwardly", "downwardly", "vertical", "horizontal" and the like are used herein for the purpose of explanation only unless specifically indicated otherwise.

As will be appreciated by one of skill in the art, embodiments of the present inventive concept may be embodied as a method, system, data processing system, or computer program product. Accordingly, the present inventive concept may take the form of an embodiment combining software and hardware aspects, all generally referred to herein as a "circuit" or "module." Furthermore, the present inventive concept may take the form of a computer program product on a non-transitory computer usable storage medium having computer usable program code embodied in the medium. Any suitable computer readable medium may be utilized including hard disks, CD ROMs, optical storage devices, or other electronic storage devices.

Computer program code for carrying out operations of the present inventive concept may be written in an object oriented programming language such as Matlab, Mathematica, Java, Smalltalk, C or C++. However, the computer program code for carrying out operations of the present inventive concept may also be written in conventional procedural programming languages, such as the "C" programming language or in a visually oriented programming environment, such as Visual Basic.

Certain of the program code may execute entirely on one or more of a user's computer, partly on the user's computer, as a stand alone software package, partly on the user's computer and partly on a remote computer or entirely on the remote computer. In the latter scenario, the remote computer may be connected to the user's computer through a local area network (LAN) or a wide area network (WAN), or the connection may be made to an external computer (for example, through the Internet using an Internet Service Provider).

The inventive concept is described in part below with reference to flowchart illustrations and/or block diagrams of methods, devices, systems, computer program products and data and/or system architecture structures according to embodiments of the inventive concept. It will be understood that each block of the illustrations, and/or combinations of blocks, can be implemented by computer program instructions. These computer program instructions may be provided to a processor of a general-purpose computer, special purpose computer, or other programmable data processing apparatus to produce a machine, such that the instructions, which execute via the processor of the computer or other programmable data processing apparatus, create means for implementing the functions/acts specified in the block or blocks.

These computer program instructions may also be stored in a computer readable memory or storage that can direct a computer or other programmable data processing apparatus to function in a particular manner, such that the instructions stored in the computer-readable memory or storage produce an article of manufacture including instruction means which implement the function/act specified in the block or blocks.

The computer program instructions may also be loaded onto a computer or other programmable data processing apparatus to cause a series of operational steps to be performed on the computer or other programmable apparatus to produce a computer implemented process such that the instructions which execute on the computer or other programmable apparatus provide steps for implementing the functions/acts specified in the block or blocks.

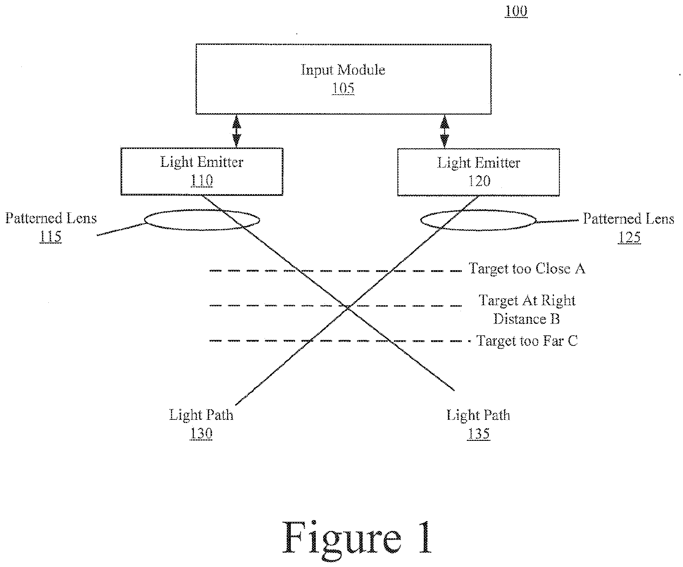

As discussed above, during the imaging process, the distance from the target (sample) to the camera lens needs to be within a certain range to ensure quality of the image, sufficient illumination and size of the field of view. This is distance is referred to herein as the "object distance." Furthermore, it is also useful to know the dimensions of the target region. Accordingly, some embodiments of the present inventive concept provide this information in a non-invasive manner. Some embodiments discussed herein measure the dimensions of the target region without contacting the target while ensuring the proper object distance during the imaging procedure as will be discussed herein with respect to Figures 1 through 10 below.

Referring first to Figure 1, a system 100 for imaging in accordance with some embodiments of the present inventive concept will be discussed. As illustrated in Figure 1, the imaging system includes an input module 105, first and second light emitters 110 and 120, respectively, both having corresponding patterned lenses 115 and 125. The first and second light emitters may be, for example, laser emitters, light emitting diodes (LEDs) or other light source without departing from the scope of the present inventive concept. The input module 105 may be used to control the system including the light emitters 110 and 120. Although embodiments of the present inventive concept are discussed herein with respect to the use of two light emitters, embodiments of the present inventive concept are not limited to this configuration. For example, three or more light emitters may be used without departing from the scope of the present inventive concept.

Furthermore, the system illustrated in Figure 1 may be used in combination with any imaging system without departing from the scope of the present inventive concept. As discussed above, embodiments of the present inventive concept may be used in any format of clinical imaging, which includes both surgical imaging (usually an in-patient application) and other out-patient imaging procedure (non-surgical application) without departing from the scope of the present inventive concept.

In some embodiments, the first and second light emitters 110 and 120, respectively, may be low power (mW level) laser emitters having wavelengths in a range from about 350nm to about 1100 nm. Each laser can project a pattern on an object plane, for example, a crosshair pattern as will be discussed further below. It will be understood that embodiments of the present inventive concept are not limited to a crosshair pattern and that any pattern that lends itself to embodiments discussed herein may be used without departing from the scope of the present inventive concept. For example, the patterns may be one horizontal line and one vertical line, which when configured correctly may look like a cross. The pattern may be etched on the lens (115 and 125) associated with the light emitters 110 and 120. Although embodiments discussed herein discuss etching as the method of placing the pattern on the lens 115/125, embodiments of the present inventive concept are not limited thereto. For example, the pattern may be tattooed on the lens using ink without departing from the scope of the present inventive concept.

When the first and second light emitters are aimed at the object plane at a certain angle, at the proper object distance from the target, the center of the two cross hair patterns may overlap on top of each other, which indicates to the user that the camera lens is at the proper object distance from the sample, position B on Figure 1. As further illustrated in Figure 1, when the two patterns do not properly overlap, the user will know to adjust the distance from the camera lens to the target as the positions A (target too close) and C (target too far) will be clear by how the patterns interact with one another as will be discussed further below.

Although illustrated and discussed herein as a point that overlaps in the patterns, embodiments of the present inventive concept are not limited to this configuration. The overlapping pattern may take many forms other than a point, for example, a line, a shape and the like.

As will be discussed further herein, each pattern, for example, crosshairs, may include tick marks labeled in units of distance, for example, cm/inch, on both horizontal and vertical lines. When the right distance is obtained by overlapping the two patterns as discussed above, the tick marks on the crosshairs are in the right scale and will convey the dimensions of the target in the specific unit of distance.

Thus, as discussed above, a light source may generate a beam, for example, a laser beam with a low power, for example, several milliwatts, from the first and second light emitters 110 and 120, respectively. After the first and second beams 130 and 135, respectively, go through the first and second patterned lenses 115 and 125, respectively, a specific pattern, such as cross hair pattern can be projected onto a target plane. If the two beams are aimed at a proper angle, the centers of the projected patterns will overlap on top of each other (position B) on a target plane located at certain distance in front of the laser emitters, as illustrated in Figure 1. If the target plane is too close (position A) or too far (position C) away from the laser emitters, the centers of projected patterns will not overlap on top of each other.

Using the light emitters and the patterned lenses as discussed above embodiments of the present inventive concept provide distance marking and dimension measuring during real time clinical imaging, which includes both surgical imaging and other out-patient non-surgical imaging. The dimensions indicated on the patterns, as discussed above, can be obtained without contacting the subject and can be captured for the record by the camera.

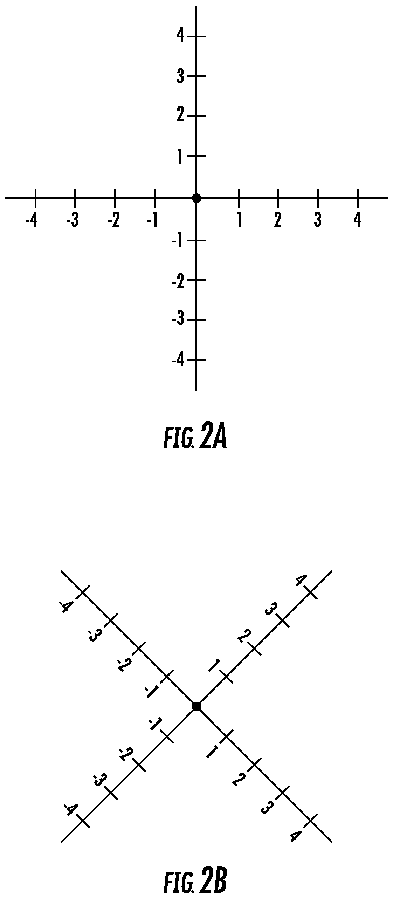

Referring now to Figures 2A and 2B, example crosshair patterns in accordance with some embodiments of the present inventive concept will be discussed. As discussed above, embodiments of the present inventive concept are not limited to use of crosshair patterns and these are discussed herein as examples only. As illustrated in Figure 2A, a cross hair pattern having tick marks (1-4) on each of the axes may be projected onto the target plane by one of the light emitters. Similarly, as illustrated in Figure 2B a rotated cross hair pattern having tick marks on each of the axes may be projected onto the target plane by a second one of the light emitters.

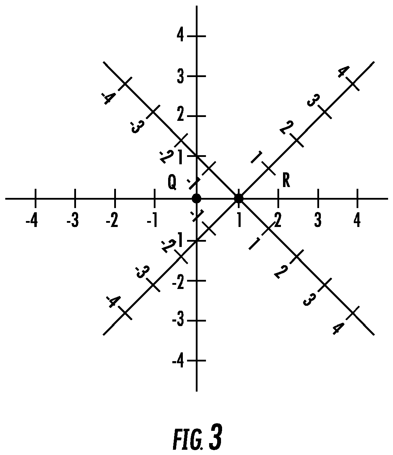

Then, as illustrated in Figure 3, the projected cross hair patterns illustrated in Figures 2A and 2B may overlap and provide an indication as to when the target plane is at a proper distance from the camera lens. Figure 3 illustrates a situation where the target plane is not at the right distance as the center points Q and R of the two cross hair patterns of Figures 2A and 2B do not directly overlap. Thus, the tick marks on the axes will not be properly scaled to reflect the dimensions of the target.

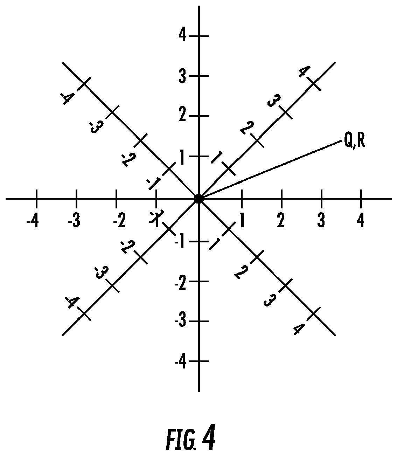

The crosshair patterns of Figure 4, on the other hand, illustrate the projected cross hair patterns of Figures 2A and 2B when the target plane is at the proper distance from the camera, such that the center points of the two cross hair patterns directly overlap. Thus, the tick marks on the axes will properly reflect the real dimensions of the target.

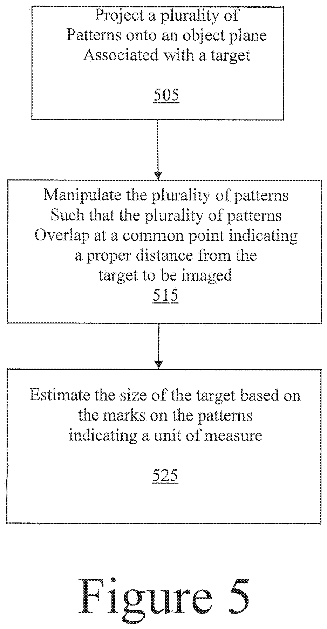

Referring now to Figure 5, a flowchart illustrating operations of a system in accordance with embodiments discussed herein will be discussed. As illustrated in Figure 5, operations of a method for determining parameters during a clinical procedure begin at block 505 by projecting a plurality of patterns onto an object plane associated with a target to be imaged. The plurality of patterns may be manipulated such that the plurality of patterns overlaps at one of a common point, line or other geometry indicating a proper object distance from the target to be imaged (block 515).

In some embodiments, the plurality of patterns may include marks indicating a unit of measure indicating dimensions of the target to be imaged. Thus, target dimensions may be obtained without contacting the target and may be stored by capturing the image with the camera. In some embodiments, the plurality of patterns may be crosshair patterns projected onto the object plane. As discussed above, each of the crosshair patterns may have tick marks on the axes indicating a unit of measure. Thus, in these embodiments, the crosshair patterns may be manipulated such that center points of each directly overlap indicating the proper object distance from the target. The size/dimension of the target may be determined based on the tick marks or markings on the pattern (block 525). Methods in accordance with embodiments discussed herein may be performed during one of a clinical and/or surgical imaging procedure in real time.

In some embodiments, the images are projected using laser emitters having wavelengths of from about 350nm to about 1000nm. The method may be non-invasive and performed in real time.

It will be understood that although embodiments of the present inventive concept are discussed with respect to crosshair patterns, embodiments of the present inventive concept arc not limited to this configuration. Any pattern that lends itself to the present inventive concept may be used without departing from the scope of the present inventive concept. Similarly, although only two light emitters (laser emitters) and associated patterns are discussed herein, embodiments are not limited to the use of only two emitters.

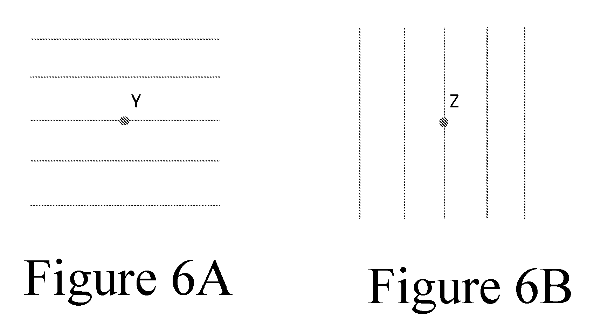

Referring now to Figures 6A through 10, an example of images obtained using distance markers and optical ruler function in accordance with embodiments of the present inventive concept will be discussed. Referring first to Figures 6A and 6B, a system including two light emitters, for example, the system illustrated in Figure 1, is used to generate the horizontal and vertical patterns illustrated therein, respectively. Each of the patterns includes a dot Y, Z in the middle thereof.

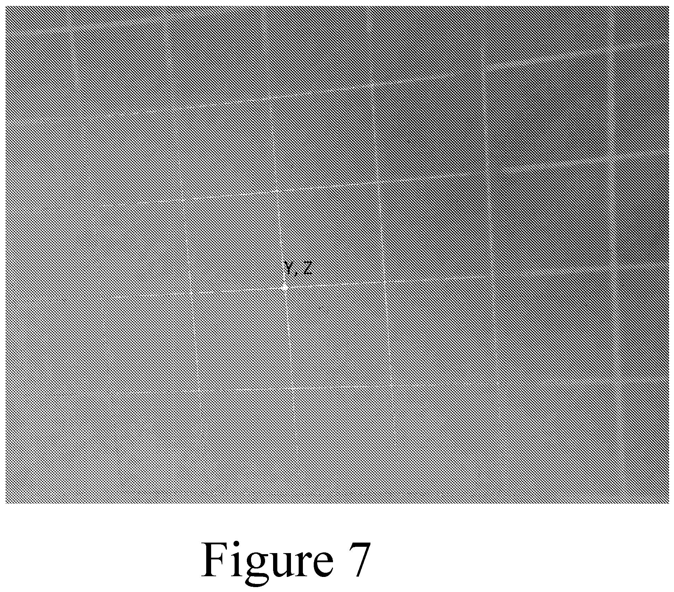

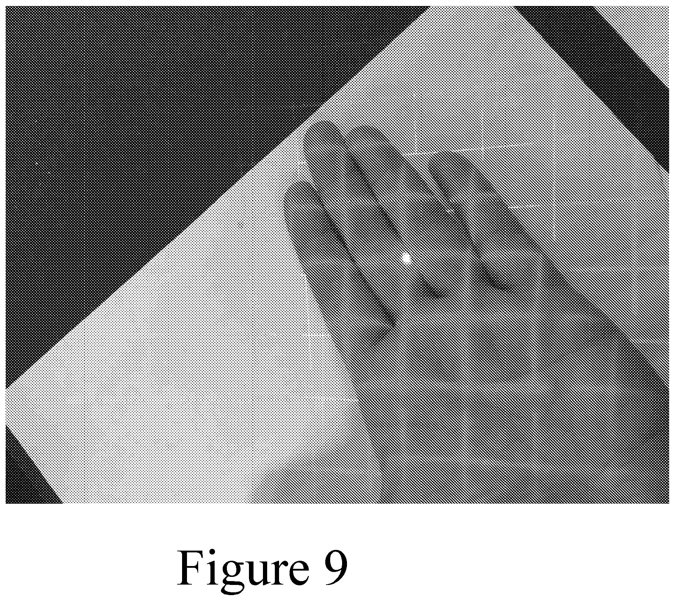

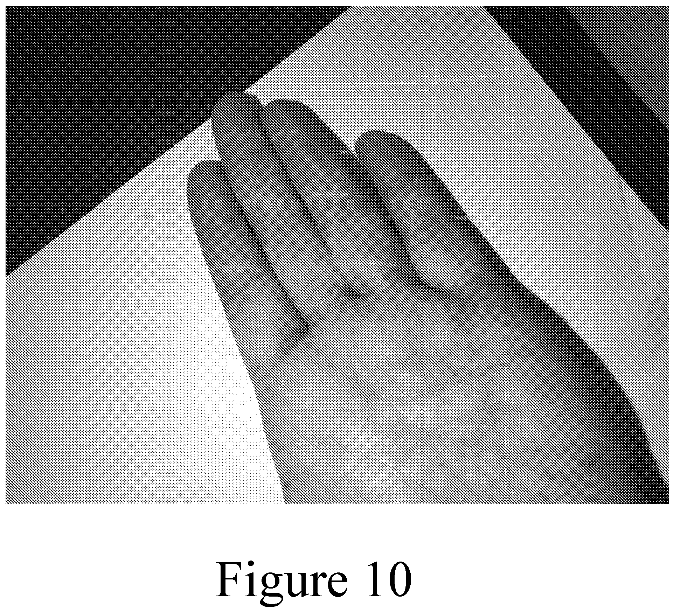

When a correct distance from the target (sample) to the camera (object distance) is achieved, it will appear as if only a single dot is present as dots Y, Z will overlap as shown in Figure 7. Thus, when the correct object distance is achieved, two dots are overlaying on top of each other as show in Figure 7. In embodiments illustrated in Figure 7, each block is calibrated to a particular size, such as one inch by one inch, to serve as two dimensional ruler in order to estimate a size of the target. Embodiments of the present inventive concept illustrated in Figures 9 and 10 that the wavelength and the pattern of the light emitters can be carefully selected to avoid interference and noise from other light sources, such as LEDs, room lights and the like. Thus, the ruler function according to embodiments of the present inventive concept discussed herein is multi dimensional, for example, two-dimensional, x and y.

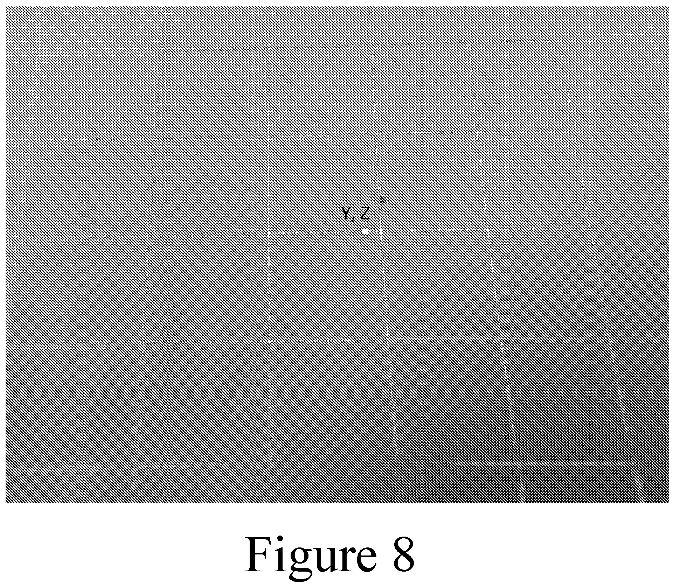

Similarly, when the object distance is not right, the two dots Y, Z are not overlaying on top of each other as shown in Figure 8.

In embodiments illustrated in Figures 6-10, the measuring function can be achieved using the optical ruler *i.e.* the blocks illustrated in the figures. In some embodiments, each block may represent a fixed distance. As shown in Figure 9, when a target, *i.e.* a human hand, is placed in the emitter beams, the grid pattern is shown on the hand. Figure 9 illustrates embodiments where a room light is on and an LED light is off and Figure 10 illustrates embodiments where both the room light and the LED lights are on. In other words, the wavelength and pattern can be selected so the functionality is not affected by the ambient light.

It will be understood that the grid pattern illustrated in Figures 6 through 10 is provided as an example only. The pattern may be changed without departing from the scope of the present inventive concept. For example, the thickness of the lines and the size of the blocks can be adjusted.

As briefly discussed above, some embodiments of the present inventive concept provide method, systems and computer program products for determining an optimal object distance from a target region as well as the dimensions of the target itself. Embodiments provide the information based on overlapping patterns projected on an object plane using light emitters as discussed above.

Some embodiments of the present invention may be understood by reference to the following numbered clauses:
1. A method for determining parameters during a clinical procedure, the method comprising:
   projecting a first pattern from a light emitter onto an object plane associated with a target to be imaged;
   projecting a second pattern from the light emitter onto the object plane associated with the target to be imaged; and
   manipulating the first and second patterns such that the first and second patterns overlap at one of a common point, line or other geometry indicating a proper object distance from the target to be imaged.
2. The method of Clause 1:
   wherein projecting the first and second patterns comprises projecting the first and second patterns having marks indicating a unit of measure indicating dimensions of the target to be imaged; and
   wherein the units of measure functions in at least two dimensions.
3. The method of Clause 2, wherein projecting the first and second patterns comprises projecting first and second crosshair patterns onto the object plane, each of the crosshair patterns having tick marks on the axes indicating a unit of measure.
4. The method of Clause 3, wherein manipulating further comprises manipulating the first and second crosshair patterns such that center points of each directly overlap indicating the proper object distance from the target.
5. The method of Clause 2, wherein the method is performed during one of a clinical and/or surgical imaging procedure in real time.
6. The method of Clause 1, wherein the light emitter comprises a first light emitter that projects the first pattern onto the object plane and a second light emitter that projects the second pattern onto the object plane.
7. The method of Clause 6, wherein projecting further comprises projecting the first and second patterns onto the object plane using first and second laser emitters, respectively, each having wavelengths of from about 350nm to about 1000nm.
8. The method of Clause 1, wherein the method is non-invasive and performed in real time.
9. The method of Clause 1, wherein the object distance is a distance from the target to a camera lens.
10. The method of Clause 1, wherein a wavelength of the emitters and corresponding patterns are selected such that functionality is not affected by ambient light.
11. A system for determining parameters during a clinical procedure, the system comprising:
   a plurality of light emitters including corresponding patterned lenses, a first and a second of the plurality of light emitters being configured to project first and second patterns, respectively, onto an object plane associated with a target to be imaged; and
   an input module configured to manipulate the first and second patterns such that the first and second patterns overlap at one of a common point, line or other geometry indicating a proper object distance from the target to be imaged.
12. The system of Clause 11, wherein the plurality of light emitters comprises three or more light emitters each having a corresponding patterned lens.
13. The system of Clause 11, wherein the first and second light emitters comprise first and second laser emitters.
14. The system of Clause 11, wherein the imaging procedure is performed during one of a clinical procedure and a surgical procedure.
15. A computer program product for determining parameters during a clinical procedure, the computer program product being stored in a non-transitory computer-readable storage medium, the computer-readable program code comprising:
   a non-transitory computer-readable storage medium having computer-readable program code embodied in the medium, the computer-readable program code comprising:
   computer readable program code configured project a plurality of patterns onto an object plane associated with a target to be imaged; and
   computer readable program code configured to manipulate the plurality of patterns such that the plurality of patterns overlap at one of a common point, line or other geometry indicating a proper object distance from the target to be imaged.
16. The computer program product of Clause 15, wherein the computer readable program code configured to project a plurality of patterns comprises:
   computer readable program code to project a first pattern from a light emitter onto an object plane associated with a target to be imaged; and
   computer readable program code to project a second pattern from the light emitter onto the object plane associated with the target to be imaged.
17. The computer program product of Clause 16, further comprising computer readable program code configured to project the first and second patterns having marks indicating a unit of measure indicating dimensions of the target to be imaged.
18. The computer program product of Clause 17, further comprising computer readable program code configured to project first and second crosshair patterns onto the object plane, each of the crosshair patterns having tick marks on the axes indicating a unit of measure.
19. The computer program product of Clause 18, further comprising computer readable program code to manipulate the first and second crosshair patterns such that center points of each directly overlap indicating the proper object distance from the target.
20. The computer program product of Clause 16, wherein the light emitter comprises a first light emitter that projects the first pattern onto the object plane and a second light emitter that projects the second pattern onto the object plane.
21. The computer program product of Clause 20, further comprising computer readable program code to project the first and second patterns onto the object plane using first and second laser emitters, respectively, each having wavelengths of from about 350nm to about 1000nm.
22. The computer program product of Clause 16, wherein the object distance is a distance from the target to a camera lens.

In the specification, there have been disclosed embodiments of the inventive concept and, although specific terms are used, they are used in a generic and descriptive sense only and not for purposes of limitation. The following claims are provided to ensure that the present application meets all statutory requirements as a priority application in all jurisdictions and shall be construed as setting forth the scope of the present inventive concept.

## Claims

1. A method for determining parameters during a clinical procedure, the method comprising:
projecting a first pattern on a first patterned lens (115) from a first light emitter (110) onto an object plane associated with a target to be imaged;
projecting a second pattern on a second patterned lens (125) from a second light emitter (120) onto the object plane associated with the target to be imaged; and
manipulating the first and second patterns such that the first and second patterns corresponding to the first and second patterned lenses (115, 125), respectively, overlap at one of a common point, line or other geometry indicating a proper object distance from the target to be imaged.

2. The method of Claim 1:
wherein projecting the first and second patterns on the first and second patterned lenses, respectively, comprises projecting the first and second patterns having marks indicating a unit of measure indicating dimensions of the target to be imaged; and
wherein the units of measure functions in at least two dimensions.

3. The method of Claim 2, wherein projecting the first and second patterns comprises projecting first and second crosshair patterns onto the object plane, each of the crosshair patterns having tick marks on the axes indicating a unit of measure.

4. The method of Claim 3, wherein manipulating comprises adjusting distances from the patterned lenses (115, 125) to the target such that center points of each of the first and second crosshair patterns directly overlap indicating the proper object distance from the target.

5. The method of Claim 2, wherein the method is performed during one of a clinical and/or surgical imaging procedure in real time.

6. The method of Claim 1, wherein the first pattern is one of etched and tattooed on the first patterned lens (115) and wherein the second pattern is one of etched and tattooed on the second patterned lens (125).

7. The method of Claim 6, wherein projecting further comprises projecting the first and second patterns onto the object plane using first and second laser emitters, respectively, each having wavelengths of from about 350nm to about 1000nm.

8. The method of Claim 1, wherein the method is non-invasive and performed in real time.

9. The method of Claim 1, wherein the object distance is a distance from the target to a camera lens.

10. The method of Claim 1, wherein a wavelength of the emitters and corresponding patterns are selected such that functionality is not affected by ambient light.

11. A system for determining parameters during a clinical procedure, the system comprising:
a first light emitter (110) and a first patterned lens (115) configured to project a first pattern onto an object plane associated with a target to be imaged and a second light emitter (120) and a second patterned lens (125) configured to project a second pattern onto the object plane associated with the target to be imaged; and
an input module (105) configured to manipulate the first and second patterns such that the first and second patterns overlap at one of a common point, line or other geometry indicating a proper object distance from the target to be imaged.

12. The system of Claim 11, comprising three or more light emitters each having a corresponding patterned lens.

13. The system of Claim 11, wherein the first and second light emitters (110, 120) comprise first and second laser emitters.

## Patentansprüche

1. Verfahren zum Bestimmen von Parametern während eines klinischen Verfahrens, wobei das Verfahren Folgendes beinhaltet:
Projizieren eines ersten Musters auf einer ersten gemusterten Linse (115) von einem ersten Lichtemitter (110) auf eine mit einem abzubildenden Ziel assoziierte Objektebene;
Projizieren eines zweiten Musters auf einer zweiten gemusterten Linse (125) von einem zweiten Lichtemitter (120) auf die mit dem abzubildenden Ziel assoziierte Objektebene; und
Manipulieren des ersten und zweiten Musters, so dass sich das erste und zweite Muster, die der ersten bzw. zweiten gemusterten Linse (115, 125) entsprechen, an einem/r gemeinsamen Punkt, Linie oder einer anderen Geometrie überlappen, die einen geeigneten Objektabstand von dem abzubildenden Ziel anzeigt.

2. Verfahren nach Anspruch 1:
wobei das Projizieren des ersten und zweiten Musters auf der ersten bzw. zweiten gemusterten Linse das Projizieren des ersten und zweiten Musters mit Markierungen beinhaltet, die eine Maßeinheit anzeigen, die die Abmessungen des abzubildenden Ziels anzeigt; und
wobei die Maßeinheiten in mindestens zwei Dimensionen funktionieren.

3. Verfahren nach Anspruch 2, wobei das Projizieren des ersten und zweiten Musters das Projizieren eines ersten und zweiten Fadenkreuzmusters auf die Objektebene beinhaltet, wobei jedes der Fadenkreuzmuster Teilstriche auf den Achsen aufweist, die eine Maßeinheit anzeigen.

4. Verfahren nach Anspruch 3, wobei das Manipulieren das Einstellen von Abständen von den gemusterten Linsen (115, 125) zum Ziel beinhaltet, so dass sich Mittelpunkte des ersten und zweiten Fadenkreuzmusters jeweils direkt überlappen, was den richtigen Objektabstand vom Ziel anzeigt.

5. Verfahren nach Anspruch 2, wobei das Verfahren während eines klinischen und/oder chirurgischen Abbildungsverfahrens in Echtzeit durchgeführt wird.

6. Verfahren nach Anspruch 1, wobei das erste Muster auf die erste gemusterte Linse (115) geätzt oder tätowiert wird und wobei das zweite Muster auf die zweite gemusterte Linse (125) geätzt oder tätowiert wird.

7. Verfahren nach Anspruch 6, wobei das Projizieren ferner das Projizieren des ersten und zweiten Musters auf die Objektebene mittels eines ersten bzw. zweiten Laseremitters beinhaltet, die jeweils Wellenlängen von etwa 350 nm bis etwa 1000 nm haben.

8. Verfahren nach Anspruch 1, wobei das Verfahren nicht-invasiv ist und in Echtzeit durchgeführt wird.

9. Verfahren nach Anspruch 1, wobei der Objektabstand ein Abstand vom Ziel zu einer Kameralinse ist.

10. Verfahren nach Anspruch 1, bei dem eine Wellenlänge der Emitter und entsprechenden Muster so gewählt wird, dass die Funktionalität durch Umgebungslicht nicht beeinträchtigt wird.

11. System zum Bestimmen von Parametern während eines klinischen Verfahrens, wobei das System Folgendes umfasst:
einen ersten Lichtemitter (110) und eine erste gemusterte Linse (115), die zum Projizieren eines ersten Musters auf eine mit einem abzubildenden Ziel assoziierte Objektebene konfiguriert sind, und einen zweiten Lichtemitter (120) und eine zweite gemusterte Linse (125), die zum Projizieren eines zweiten Musters auf die mit dem abzubildenden Ziel assoziierte Objektebene konfiguriert ist; und
ein Eingabemodul (105), das zum Manipulieren des ersten und zweiten Musters konfiguriert ist, so dass sich das erste und zweite Muster an einem/r gemeinsamen Punkt, Linie oder anderen Geometrie überlappen, die einen geeigneten Objektabstand von dem abzubildenden Ziel anzeigt.

12. System nach Anspruch 11, das drei oder mehr Lichtemitter jeweils mit einer entsprechenden gemusterten Linse aufweisen.

13. System nach Anspruch 11, wobei der erste und zweite Lichtemitter (110, 120) einen ersten und einen zweiten Laseremitter umfassen.

## Revendications

1. Procédé de détermination de paramètres pendant une procédure clinique, le procédé comprenant :
une projection d'un premier motif sur une première lentille à motifs (115) à partir d'un premier émetteur de lumière (110) sur un plan objet associé à une cible à imager ;
une projection d'un second motif sur une seconde lentille à motifs (125) à partir d'un deuxième émetteur de lumière (120) sur le plan objet associé à la cible à imager ; et
une manipulation des premier et second motifs de telle sorte que les premier et second motifs correspondant aux première et seconde lentilles à motifs (115, 125), respectivement, se chevauchent au niveau d'un élément parmi un point, une ligne ou une autre géométrie communs indiquant une distance d'objet appropriée à partir de la cible à imager.

2. Procédé selon la revendication 1 :
dans lequel une projection des premier et second motifs sur les première et seconde lentilles à motifs, respectivement, comprend une projection des premier et second motifs présentant des marques indiquant une unité de mesure indiquant des dimensions de la cible à imager ; et
dans lequel les unités de mesure fonctionnent dans au moins deux dimensions.

3. Procédé selon la revendication 2, dans lequel une projection des premier et second motifs comprend une projection de premier et second motifs de pointeur sur le plan objet, chacun des motifs de pointeur présentant des marques de graduation sur les axes indiquant une unité de mesure.

4. Procédé selon la revendication 3, dans lequel une manipulation comprend un ajustement de distances des lentilles à motifs (115, 125) jusqu'à la cible de telle sorte que des points centraux de chacun des premier et second motifs de pointeur se chevauchent directement indiquant la distance d'objet appropriée à partir de la cible.

5. Procédé selon la revendication 2, dans lequel le procédé est réalisé pendant une d'une procédure d'imagerie clinique et/ou chirurgicale en temps réel.

6. Procédé selon la revendication 1, dans lequel le premier motif est un de gravé et tatoué sur la première lentille à motifs (115) et dans lequel le second motif est un de gravé et tatoué sur la seconde lentille à motifs (125).

7. Procédé selon la revendication 6, dans lequel une projection comprend en outre une projection des premier et second motifs sur le plan objet en utilisant de premier et second émetteurs laser, respectivement, chacun présentant des longueurs d'onde d'environ 350 nm à environ 1000 nm.

8. Procédé selon la revendication 1, dans lequel le procédé est non invasif et réalisé en temps réel.

9. Procédé selon la revendication 1, dans lequel la distance d'objet est une distance de la cible à une lentille de caméra.

10. Procédé selon la revendication 1, dans lequel une longueur d'onde des émetteurs et des motifs correspondants sont sélectionnés de telle sorte qu'une fonctionnalité n'est pas affectée par la lumière ambiante.

11. Système de détermination de paramètres pendant une procédure clinique, le système comprenant :
un premier émetteur de lumière (110) et une première lentille à motifs (115) configurés pour projeter un premier motif sur un plan objet associé à une cible à imager et un deuxième émetteur de lumière (120) et une seconde lentille à motifs (125) configurés pour projeter un second motif sur le plan objet associé à la cible à imager ; et
un module d'entrée (105) configuré pour manipuler les premier et second motifs de telle sorte que les premier et second motifs se chevauchent au niveau d'un élément parmi un point, une ligne ou une autre géométrie communs indiquant une distance d'objet appropriée à partir de la cible à imager.

12. Système selon la revendication 11, comprenant trois émetteurs de lumière ou plus ayant chacun une lentille à motifs correspondante.

13. Système selon la revendication 11, dans lequel les premier et deuxième émetteurs de lumière (110, 120) comprennent de premier et second émetteurs laser.
